# EUROPEAN PATENT APPLICATION

(11) **EP 4 108 277 A1**
(43) Date of publication of application: **28.12.2022**
(21) Application number: 22186392.1
(22) Date of filing: 22.07.2022
(51) Int. Cl.: A61M 5/24, A61M 5/32

(54) **SYRINGE UNIT FOR A REUSABLE INJECTION DEVICE**

(71) Applicant: Ypsomed AG, 3401 Burgdorf (CH)
(72) Inventor: Scheurer, Simon, 3006 Bern (CH); Hostettler, Patrick, 3415 Hasle (CH)
(74) Representative: Kleiner, Stefan

(57) **Abstract**

The present invention relates to a disposable syringe unit (2) for an injection device (1), adapted for releasable attachment to a reusable drive unit (3) of the injection device. The syringe unit (2) comprises a prefilled syringe comprising a reservoir and an injection needle (18), a syringe holder (30) for holding the prefilled syringe (15). The syringe unit includes further a cover member (20) guided by guiding means (31) thereby movable along the longitudinal axis relative to the syringe holder (30) between a covering position in which the injection needle (18) is covered and a retracted position in which the injection needle (18) protrudes from the cover member (20) and a cap (10) releasably attached to a distal end of the cover member (20) or the syringe holder (30). Apart from the prefilled syringe the syringe unit (30) comprises in total not more than three separate parts.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from an injection device comprising a reusable drive unit and a disposable syringe unit configured for releasable attachment to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism in order to move the plunger of the syringe upon actuation of the autoinjector. The drive mechanism typically comprises a source of drive, such as a motor or strong spring for moving a transfer element, for example a rod, which acts on the plunger of the syringe.

For safety and hygiene reasons it is desirable that the needle does not protrude from a housing of the auto injector with the exception of the time when the needle is used for injection of a medicament. Thus, either the autoinjector moves the needle out of the housing for the injection and back into the housing after injection or the housing provides a needle guard which may be moved to unsheathe the needle for injection and which may be moved back to a needle covering position after the injection.

The majority of autoinjectors are configured as single use devices which incorporate both the syringe and the drive mechanism in the same housing. Such devices are usually disposable for hygiene reasons.

Disposable auto injectors comprising an electric actuator or an electronic control require a source of energy which is usually in the form of a battery. However, in this case, the autoinjectors should not be disposed in the regular waste, but have to be subjected to special disposal or to recycling, which is an additional burden to the patient. Further, disposing a battery, motor and/or electronics after a single use is a waste of resources and increases the costs of the auto injector.

In order to account for a need to handle and dispose the autoinjector parts differently semi-reusable autoinjectors have been developed. Such autoinjectors typically comprise a reusable drive unit as well as a disposable syringe unit which may be releasably coupled to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for the upcoming injection.

WO 2010/046569 discloses a reusable autoinjector, where a syringe unit is to be coupled to a drive unit by a bayonet connection. Once a cam of the drive unit is inserted in a slot in the syringe unit housing a cover sleeve is unlocked and can be moved to unsheathe the needle for an injection. The syringe unit includes a unit-housing, a shield, a syringe holder and two springs biasing the shield to a covering position.

WO 2021/069106 discloses an autoinjector comprising a disposable syringe unit and a reusable drive unit. Apart from a syringe barrel with a rigid needle shield the syringe unit comprises a syringe holder, a syringe holder support tube, syringe holder arms, a cassette housing, a sensor sleeve, an outer shield tube and a cassette cap mounted on a distal end of the syringe unit.

WO 2012/145685 discloses further a reusable autoinjector with a disposable or single-use cassette and a reusable drive unit. The cassette includes an outer housing, an inner sleeve, a shield remover, a movable cover and a lock cap locking the syringe to the inner sleeve.

### DESCRIPTION OF THE INVENTION

It is an objective of the invention to provide a simplified manufacturing, handling and to reduce waste for semi-reusable injection devices.

This objective is achieved by a disposable syringe unit or an injection device according to the independent claims. Preferred embodiments are evident from the dependent claims.

The invention relates to a disposable or single-use syringe unit (or reservoir unit) for an injection device configured for releasable attachment to a reusable drive unit of the injection device. According to the invention the syringe unit consists of:
- a prefilled syringe comprising a reservoir and an injection needle (18) permanently connected to the reservoir and optionally covered by a needle shield in a shipping state;
- a syringe holder (30) for holding the prefilled syringe (15), the syringe holder (30) comprising guiding means (31) and defining a longitudinal axis;
- a cover member (20) guided by the guiding means (31) thereby movable along the longitudinal axis relative to the syringe holder (30) between a covering position in which the injection needle (18) is covered and a retracted position in which the injection needle (18) exposes and protrudes from the cover member (20);
- a cap (10) releasably attached to a distal end of the cover member (20) or the syringe holder (30); Apart from the prefilled syringe (including syringe elements) the syringe unit (30) comprises in total not more than three separate parts.

Syringe elements may be an injection needle in separately connected to a glass syringe barrel, a piston movably arranged inside the syringe barrel and a needle shield directly mounted onto the metallic injection needle in an unused or shipping state.

That means according to the invention the syringe unit comprises or consists of in total at maximum three separate parts (prefilled syringe with syringe elements not counted) made of plastic or metal or both plastic and metal, preferably the three parts are separately produced.

As apart from the prefilled syringe the syringe unit comprises not more than three parts the manufacturing, the assembly, the handling and storage of the syringe unit is simplified compared to syringe units with more parts. A reduced amount of parts can thus help to reduce the overall production costs and reduce the complexity of the syringe unit.

Besides that, the amount of discarded parts and thus the waste of a reusable injection device concept can be reduced which is advantageous from an environmental perspective.

The disposable syringe unit is part of the semi-disposable or semi-reusable injection device. The syringe unit is hence not operational without a reusable drive unit of the injection device. To prepare the injection device for an injection the user has to attach or to connect the syringe unit to a drive unit. The drive unit may include drive means adapted to dispense the liquid drug from the syringe hold inside the syringe unit once the syringe unit is attached to the drive unit.

The injection device may be a manual or an automatic injection device. Automatic devices or autoinjectors typically include automatic drive means such as an elastic element (for example a pretensioned spring) or an electric motor to drive a dispensing member to dispense the liquid drug form the syringe. Manual injection devices include a manual drive such as a dispensing button that has to be pressed by the user to move the dispensing member in dispensing direction.

The cover member or needle cover is movable along the longitudinal axis and guided by the syringe holder. The cover member may be implemented as shield or sleeve or sleeve-shaped element adapted to cover or envelop a needle of a prefilled syringe mounted inside the syringe holder. The movement of the needle cover allows to switch between a safety state in which the needle is covered and the needle does not protrude from a device housing and an injection state in which the needle is uncovered and exposes or protrudes from the housing.

In a preferred embodiment the cover member is the outermost part of the disposable syringe unit. That means the syringe unit does not comprise any outer housing or shell but only the movable cover member. This provides for a simple design.

The syringe holder, the cover member and the cap are preferably plastic parts. The term "separate" means that before assembly the parts can be handled independently from each other and are preferably produced separately and independently from other parts. That does not exclude that the parts may be connected upon assembly of the syringe unit. The parts may be connected to each other, for example, by a snap-fit or a thread.

As stated above the prefilled syringe with its possibly present needle shield (often named as rigid needle shield, RNS) is not counted as the syringe is usually inserted and fixed to the pre-assembled syringe unit at a later stage. Likewise, a possibly present label, sticker or tag attached to an outside surface of one of the syringe unit parts does not count as a separate part.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In the claims, the word "comprising" does not exclude other elements or steps, and the indefinite article "a" or "an" does not exclude a plurality. For example "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

Preferably, the syringe holder, the cover sleeve and the cap are individually injection-molded thermoplastic plastics parts. That means the syringe holder, cover sleeve and the cap are not made from an elastomeric material. In comparison a piston or bung arranged inside the glass barrel of the syringe may be made of an elastomeric material like rubber. Such a piston or bung is a syringe element.

The injection-molded parts allow for a cost-effective production of the syringe unit parts in particular in case of a high volume production.

The syringe is preferably non-movable arranged within the syringe holder after assembly. That means during an injection the syringe does not move relative to the syringe holder meaning that the user has to insert or penetrate the injection needle into the injection site by a manual insertion force.

The syringe includes usually a cylindrical barrel made of glass and an injection needle made of steel inseparably connected to the barrel. Furthermore, the syringe may include the rigid needle shield which is directly mounted onto the injection needle upon production of the syringe and which prevents access to the needle before removal of the rigid needle shield.

The syringe with the barrel, the injection needle and the rigid needle shield are thus produced as a unit which can be mounted inside the syringe holder.

In a preferred embodiment apart from the syringe with its syringe elements (piston, needle, rigid needle shield directly mounted on the needle in a shipping state) the syringe unit includes exclusively plastic parts and more preferably the syringe unit includes exclusively injection molded thermoplastic plastic parts (apart from the syringe and its syringe elements). That means the syringe unit is devoid of any metal parts such as springs, clamps or the like. This facilitates the production and contributes to a cost-efficient design.

Optionally, the syringe holder includes at least one radially protruding flexible arm with a free end portion and wherein the cover member includes at least one stop surface and wherein the free end portion is adapted to engage the stop surface to hold the cover member in the covering position and thus preventing a movement of the cover member out of the covering position towards a retracted position. That means the arm allows for a reliable holding of the cover member in the covering position in which it covers the injection needle. Furthermore, by a movement of the arm or of the free end portion the cover member may be unlocked and thus allowing a quick unlock of the cover member such that it can be retracted to uncover the injection needle.

The stop surface may be arranged, for example, on a ledge or protrusion and is preferably arranged inside a cover member shell or cover member shell.

The at least one arm is in a preferred embodiment integrally formed in the syringe holder. That means the arm is monolithic with the syringe holder. That allows for an efficient production and handling as the syringe holder and the arm can be produced by one single injection-molded plastic part.

Advantageously, the end portion of the at least one arm engages the at least one stop surface in a non-deflected position of the arm and the end portion is disengaged from the ledge in a deflected state of the arm thereby allowing the cover member to be moved out of the covering position towards a retracted position in which the injection needle is uncovered or exposes. The deflectable arm allows for a quick and reliable lock or unlock of the cover member.

In a preferred embodiment the cover member includes a machine-readable code attached on a surface of the cover member. The code comprises syringe unit specific identification information. An external code reader may read the information from the code and based on read identification information the syringe unit and preferably the drug inside the syringe may be identified. The identification information may include data about the syringe, the drug, a volume, and an expiration date or injection instruction for the drug contained inside the syringe.

The external code reader may be an external device or it may be in the drive unit and adapted to read the code once the syringe unit is attached to the drive unit.

The machine-readable code may be implemented in form of optical code or a code readable based on electromagnetic fields. Examples for an optical code are an Object Identifier code (OID), QR codes, bar codes, color codes and optical patterns. Examples for codes based on electromagnetic fields are a radio-frequency identification (RFID) tag or near-field communication (NFC) tags.

The code may be attached on an outside surface of the cover member, for example, by means of a label or sticker including the code. Alternatively, the code may be on an inside surface or integrally formed in a wall of the cover member as electromagnetic tag or chip.

The cover member is a sleeve in a preferred embodiment. The syringe holder is then concentrically arranged inside the sleeve. Therefore, the cover sleeve is movable along the longitudinal axis outside and relative to the syringe holder if the cover member is not blocked or hold in the covering position. The sleeve form allows for a compact design.

Preferably, the cover sleeve includes a first radial opening or an opening. The arm of the syringe holder is thus accessible from the outside through the first opening. Therefore, the arm can be actuated through the opening to move or release the arm which in turn allows to unlock the cover member from the covering position or to lock the cover member in the covering position, respectively.

Furthermore, the syringe holder includes preferably a radially extending protrusion or rim for holding the syringe unit by the reusable unit when the syringe unit is attached or connected to the drive unit. The sleeve includes preferably a second radial opening wherein the protrusion or rim is accessible from the outside through the second opening.

Thus, the syringe unit may be held by a clamp or holding arms extending through the second opening in the cover member sleeve.

The guiding means of the syringe holder includes a longitudinal groove or a longitudinal spline on an outside surface and the cover sleeve includes on an inside surface the other of the longitudinal groove or the longitudinal spline. The cover sleeve is thus reliably guided by the spline and groove and can be moved along the longitudinal axis.

The spline may be implemented as protrusion or ledge or as swallowtail. The guiding means may be implemented alternatively as rail and clamp adapted to engage the rail.

The invention relates further to an injection device comprising the disposable syringe unit as described above and the reusable drive unit. The latter may include a plunger rod and a drive for moving the plunger rod in a dispensing direction to dispense the drug from the syringe. The syringe unit is releasably attachable to the drive unit.

As mentioned above the injection device may be a manual injection device requiring a user force to drive the plunger rod to dispense the drug or alternatively the injection device may be an automatic injection device including an automatic drive, for example, in form of a biased spring member or an electric motor.

The drive unit may further comprise flexible holding arms engaging the radial extending protrusion or rim of the syringe holder if the syringe unit is attached to the drive unit. The flexible holding arms allow for a releasably attachment of the syringe unit to the drive unit.

In a preferred embodiment the cover member may be locked to the syringe holder in the covering position, and the cover member may be unlocked by relative movement between the syringe unit and the drive unit. The syringe unit may be moved manually by a user or automatically by the automatic drive of the drive unit.

Preferably, the injection device is a semi-reusable (or semi-disposable) autoinjector. The automatic drive may comprise an electric motor adapted to drive the plunger rod to dispense the drug from the syringe.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of a semi-reusable autoinjector according to the invention;
- Fig.2: depicts an exploded view of the syringe unit of the autoinjector from figure 1;
- Fig.3: depicts a sectional view of the drive unit of the autoinjector;
- Fig. 4: depicts the drive unit with an inserted syringe unit and
- Fig. 5: depicts a sectional view of the autoinjector where the syringe unit is locked to the drive unit.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

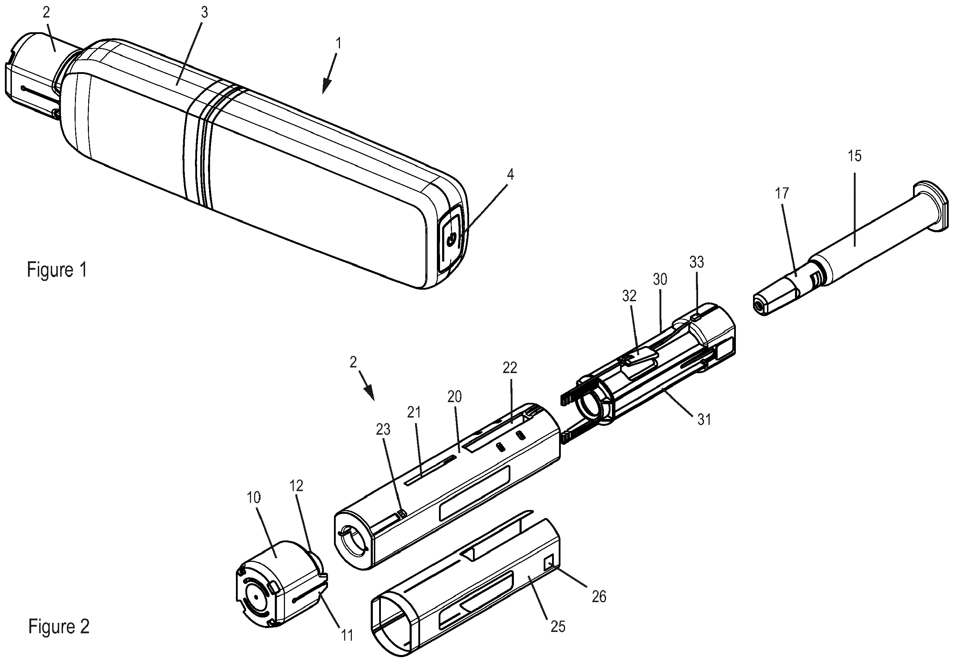

In the present description the term "distal" refers to the side where the injection needle is located. This is on the left hand side in the figures 1 to 5. The term "proximal" refers to the opposite side or rear end of the device and is on the right hand side in the figures.

Figure 1 shows a perspective view of a drug delivery device of a first embodiment of the invention in form of a semi-reusable autoinjector 1. The autoinjector 1 comprises a disposable syringe unit 2 and a reusable drive unit 3. As shown in figure 1 the drive unit 3 has an elongated housing defining a longitudinal axis and comprises a button 4 at its proximal end.

In the following the structural features of the syringe unit and the drive unit will be descripted in detail. Subsequently, the function of the semi-reusable autoinjector 1 will be explained.

Figure 2 depicts an exploded view of the syringe unit 2. As shown in figure 2 the syringe unit 2 includes a syringe holder 30, a cover sleeve 20 coaxially arranged around the syringe holder 30 and movable relative to the syringe holder along the longitudinal axis, a label 25 wrapped around the cover sleeve 20, a cap 10 and a prefilled syringe 15 comprising a liquid drug and a rigid needle shield 17 on the injection needle. The syringe holder 30, the cover sleeve 20 and the cap 10 are individually injection-molded plastic parts.

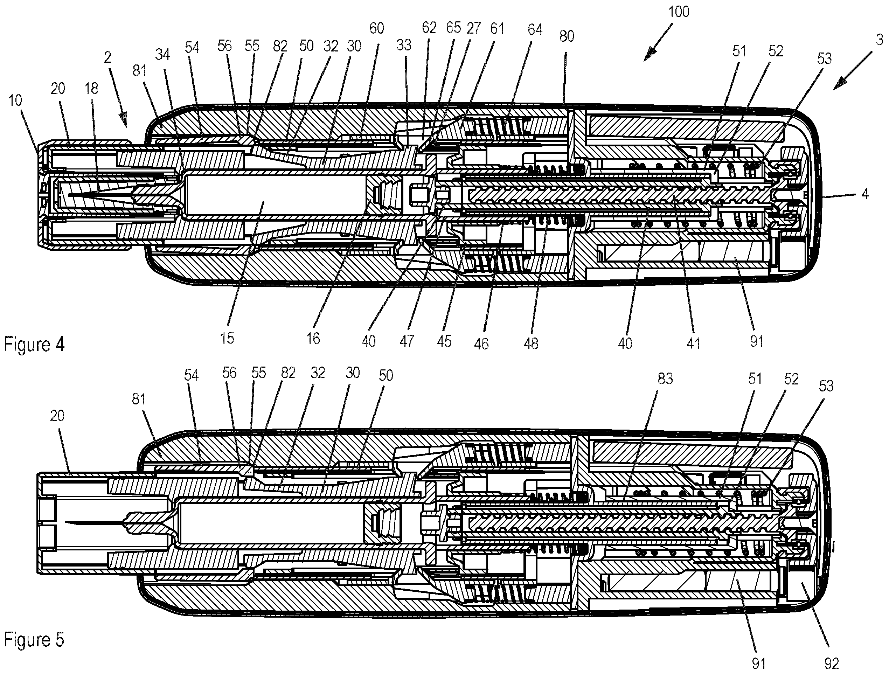

The prefilled syringe 15 with a barrel made of glass is rotationally and axially fixed inside the sleeve-shaped syringe holder 30. For that purpose, the syringe holder 30 includes clamping elements (not shown) pressing on an outer surface of the syringe barrel and a distal shoulder of the syringe abuts a bearing surface 34 (see figure 4) inside the syringe holder 30. As shown in figure 2 the syringe holder 30 further features longitudinal guiding rails 31 on its outside for the cover sleeve 20 such that the cover sleeve 20 can be shifted exclusively in the longitudinal direction relative to the syringe holder 30. Furthermore, the syringe holder 30 comprises two radially deflectable lock elements in form of locking arms 32 arranged on opposite sides of the syringe holder 30. In an initial state the locking arms 32 are in a non-deflected state and in a locking position and prevent a movement of the cover sleeve 20 relative to the syringe holder 30 in a proximal direction. Hence, the cover sleeve 20 shields or covers the injection needle of the syringe 15 in a covering position. For that purpose, a free end of the locking arms 32 abut stop surfaces (not shown) inside the cover sleeve 20. As will be descripted below once the locking arms 32 are deflected radially inwards the cover sleeve 20 can be pushed proximally. The syringe holder 30 further includes two oppositely arranged and radially protruding ledges 33 at a proximal end of the syringe holder 30 for engagement with clamping arms 61 (figure 4) inside the drive unit 3.

The cover sleeve 20 has a non-circular cross section in a plane perpendicular to the longitudinal axis. That means two sides of the cover sleeve are flattened. The cover sleeve comprises a first lateral opening 21 for access to the locking arms 32 and a proximal second opening 22 for access to the ledges 33 from outside. The label 25 wrapped around the cover sleeve 20 includes a machine-readable tag (not shown) in form of a RFID or QR code 26 identifying the syringe unit 2.

At a distal end of the cover sleeve 20 the cap 10 is releasably attached by a snap-fit connection. The cap 10 includes on two sides two deflectable elements or tongues 11 and on each side a protrusion engaging in a corresponding indentation 23 in the cover sleeve 20 for the snap-fit connection. Alternatively, the cap 10 may have an ellipse shape in a plane perpendicular to the longitudinal axis wherein inside the ellipse form the protrusions engage the indentation in the cover sleeve. The ellipse can be deformed to a circular shape to release the protrusions from the indentation 23. The release of the snap-fit will be descripted below with respect to the decapping of the syringe unit 2. The cap 10 includes in its inside a sleeve-shaped holder 12 connectable to the rigid needle shield (RNS) 17 of the syringe 15 such that the RNS 17 is removed with the cap 10 if the cap is released from the needle cover.

The syringe 15 inside the syringe holder 30 has a barrel made of glass and inside the barrel a movable piston 16 (figure 4). An injection needle 18 (figure 4) is non-releasably connected to a distal end of the syringe barrel. In an unused state the injection needle 18 is covered by the RNS 17. The latter in turn is axially and radially fixedly connected to the cap 10 by the holder 12 once the syringe 15 is mounted to the syringe holder 30.

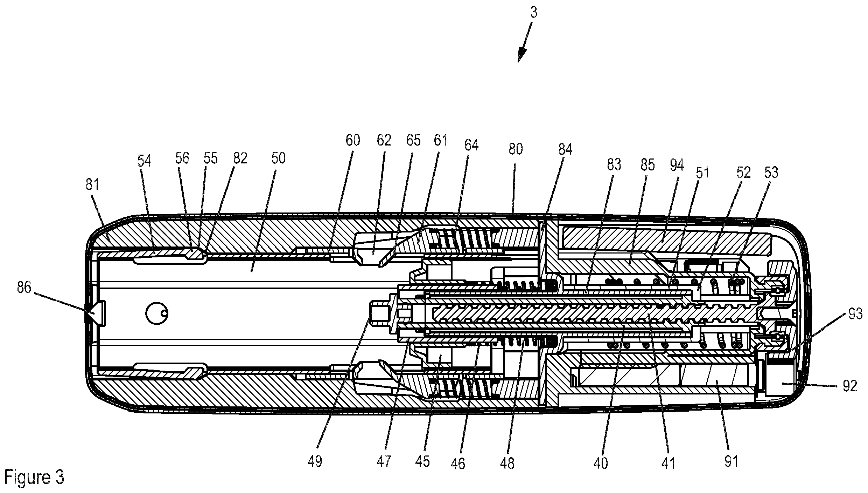

Figure 3 depicts a sectional view of a first embodiment of the drive unit 3. The cut of the sectional view runs along the longitudinal axis of the device. The drive unit 3 comprises a housing 80 including an outer housing cover and inside the cover a mechanics sleeve 83 comprising a radial wall 84 that separates the drive 3 unit in a distal portion and a proximal portion. The mechanics sleeve 83 is immovably connected to the housing 80. Inside the housing 80 and distally the separating wall 84 is a distal housing insert 81 whereas proximal the wall 84 a proximal housing insert 85 is located. The distal and proximal housing inserts 81, 85 are immovably connected to the housing 80. Furthermore, the drive unit 3 includes an opening on its distal side adapted to accommodate a proximal portion of the syringe unit 2.

Inside the distal housing portion and guided by the distal housing insert 81 a gripper sleeve 60 is arranged and movable relative to the housing 80 along the longitudinal axis. Coaxially to the gripper sleeve 60 a trigger sleeve 50 is arranged. Again inside the trigger sleeve 50 a cover sleeve connector 45 is located and biased in distal direction by a cover sleeve spring 46. The spring abuts on its distal end the cover sleeve connector 45 and on its proximal end the radial wall 84 of the mechanics sleeve 83. A sleeve-shaped syringe connector 47 is coaxially arranged inside the cover sleeve connector 45 and biased distally by a syringe connector spring 48 coaxially inside the cover sleeve spring 46 and proximally supported by the wall 84. The spring 46 ensures that the syringe connector 45 pushes the syringe 15 of an inserted syringe unit 2 in distal direction.

The proximal housing portion accommodates the proximal housing insert 85 which supports and guides a sleeve-shaped trigger sleeve connector 51 movable along the longitudinal axis, a drive assembly with an electric motor 91, a pinion 92 and a gear 93 connecting the motor 91 with a threaded rod 41, a battery 94, the treaded rod 41 and a plunger rod 40 in threadedly connected to the threaded rod 41. The proximal housing portion further accommodates an electronic module (not shown) with a controller configured to control the electronic motor 91 and providing information to the user via a display or LEDs (not shown) or a communication module (not shown). The trigger sleeve connector 51 is fixedly and immovably connected to the trigger sleeve 50 and distally pressed onto a proximal end of the plunger rod 40 by a trigger sleeve spring 53. The plunger rod 40 non-rotatably guided by the mechanics sleeve 83 and comprises on its distal end a flange 49 adapted to engage the piston 16 (see figure 4) during dispensing.

Inside the proximal housing portion is further an axially arranged detection pin (not shown) axially movable by the cover sleeve connector 45. The detection pin is connected to and interacts with a TMR position sensor. The presence of a syringe unit 2 inside the drive unit housing 80 is thus detectable via detection pin and the position sensor which provides a corresponding signal to the controller of the electronic module.

The gripper sleeve 60 includes two axially extending and deflectable clamping arms 61 having a clamp 62 at their free distal end adapted to accommodate the syringe holder ledges 33. The clamping arms 61 can be deflected radially outwards allowing the clamps 62 to snap onto the ledges 33 of the syringe holder 30 when the syringe unit is inserted into the opening in the drive unit 3. On its outside the gripper sleeve 60 comprises splines (not shown) running in corresponding longitudinal grooves (not shown) in the distal housing insert 81 guiding the gripper sleeve 60 during an axial movement relative to the housing 80. Furthermore, each clamping arm 61 provides a support for an axially aligned gripper sleeve spring 64 which biases the clamping arms 61 in a holding position.

The trigger sleeve 50 is axially guided by the gripper sleeve 60 as well as by the distal housing insert 81 and shiftable relative thereto. For this purpose, the trigger sleeve 50 includes longitudinal guiding rails (not shown) engaging longitudinal grooves (not shown) in both the distal housing insert 81 and the gripper sleeve 60. As it can be seen in figure 3 the trigger sleeve 50 incudes oppositely arranged trigger arms 54 which include on their proximal free end 56 a guiding surface or sloped contact surface 55. The trigger arms 54 are deflectable radially inwards upon contact of the contact surface 55 with a counter guiding surface or counter sloped contact surfaces 82 of the distal housing insert 81. As mentioned above the trigger sleeve 50 is further immovably connected to the trigger sleeve connector 51 in the proximal housing portion. As the trigger sleeve connector 51 is biased by the trigger sleeve spring 53 the trigger sleeve 50 is biased too in distal direction. The trigger sleeve connector 51 in turn is connected to the plunger rod via ledges 52 which engage a proximal shoulder of the plunger rod 40 such that an axial movement of the plunger rod 40 in proximal direction moves also the trigger sleeve 50 proximally.

In the state shown in figure 3 the drive unit 3 is ready to be loaded with a syringe unit 2. The controller displays the current state to the user via display by showing a message like "Syringe Unit Insertion" or via green or red LEDs.

In order to prepare the autoinjector 1 for an injection the user attaches a syringe unit 2 to the drive unit 3. The electronics in the drive unit 3 are switched from an inactive state or sleep mode to an active mode. Alternatively, the user can press the button 4 to activate the electronics.

Figure 4 depicts a sectional view of the drive unit 3 with an inserted syringe unit 2. The syringe unit 2 has been inserted with its proximal end portion into the distal opening of the drive unit housing 80. In the state shown in figure 4 the proximal ledges 33 of the syringe holder 30 are snapped into recesses of the clamps 62 of the gripper sleeve clamping arms 61. The syringe unit 2 is thus held in the drive unit 3 by the clamping arms 61. However, in the shown state in figure 4 the syringe unit 2 can still be pulled out of the drive unit housing by overcoming a holding force. Hence, if the user pulls the syringe unit 2 in a distal direction the syringe holder 30 with the syringe 15 are firstly moved in distal direction relative to the cover sleeve 20 until stop and subsequently the cover sleeve 20 is moved distally too. This brings inclined release surfaces 27 of the cover sleeve 20 into contact with inclined counter surfaces 65 of the clamping arms 61. If the cover sleeve 20 is further pulled in distal direction the clamping arms 61 are deflected radially outwards via release surfaces 27 and counter surfaces 65. The ledges 33 of the syringe holder 30 are therefore released from the clamping arms 61 and the syringe unit 2 can be removed from the drive unit 3. out of the drive unit 3 the clamping arms 61 may deflect radially outwards and release the ledges 33.

That means in the state shown in figure 4 the syringe unit 2 is held inside the drive unit 3 but not yet locked inside the drive unit 3.

Figure 5 depicts the autoinjector 1 in a locked state without cap. When the syringe unit 2 is inserted as described above the syringe unit 2 moves the detection pin and thus the position sensor detects the movement and automatically sends a signal to the controller in the electronic module which detects the presence of the syringe unit 2 inside the drive unit housing. A code reader (not shown) in form of an NFC reader or QR code reader inside the drive unit 3 reads the RFID or QR code 26 of the syringe unit 2. Subsequently, the controller of the drive unit sends the read medication information from the read code 26 to an external device to verify the drug or medication with predefined information. Alternatively, the controller compares the read medication information from the code 26 with predefined information stored in a memory inside the drive unit 2.

In case of successful medication verification the controller receives an enable signal (either from the external device or from the internal comparison). The controller then controls the electric motor 91 to rotate the threaded rod 41 to move the trigger sleeve 50 a short distance of several millimeters in proximal direction. Such a trigger sleeve 50 movement is achieved by a kinematic chain as described as follows.

The motor 91 rotates the pinion 92 and the gear 93 transfers the rotation to the threaded rod 41 which in turn moves the non-rotating plunger rod 40 in proximal direction. As the trigger sleeve connector 51 is connected via its ledges 52 to a proximal shoulder of the plunger rod 40 the latter causes the trigger sleeve connector 51 to move and thus the trigger sleeve 50 is shifted in proximal direction too.

The proximal movement of the trigger sleeve 50 brings the sloped contact surfaces 55 of the trigger arms 54 into contact with the counter sloped contact surfaces 82 of the distal housing insert 81. As the two sloped surfaces 55, 82 slide along each other the trigger arms 54 are forced to deflect radially inwards when the trigger sleeve 50 is further moved in proximal direction. The free ends 56 of the deflected trigger arms 54 in turn come into contact with the locking arms 32 of the syringe holder 30. Subsequently, the trigger arms 54 deflect the locking arms 32 radially inwards and thus move the locking arms 32 out of a locking position. As a consequence, the locking arms 32 no longer engage the stop surface of the cover sleeve 20 and thus allow for the cover sleeve 20 to move out of a covering position and move inside the housing 80 in proximal direction relative to the syringe holder 30 and relative to the drive unit housing 80.

At the same time the radially inwards deflected trigger sleeve arms 54 abut a distal stop surface (not shown) of the syringe holder 30 and thereby lock the syringe holder 30 relative to the trigger sleeve 50 such that a movement of the trigger sleeve 50 is transferred to the syringe unit 2. Thus, in this state (figure 5) the syringe unit 2 can no longer pulled out of the drive unit 3.

The controller displays the locked state to the user via display. As in this state the cap is still mounted onto the distal end of the cover sleeve 20 the next step is the decapping which may be started by pressing the button 4. The controller then drives the motor 91 to move additionally the trigger sleeve 50 a short distance of 1 to 5 mm in proximal direction and immediately the same distance back in distal direction. As the trigger arms 54 fix the syringe unit 2 relative trigger sleeve 50 the syringe unit 2 (and the gripper sleeve 60) are moved back and forth too. The proximal movement of the syringe unit 2 with the cap 10 relative to the drive unit housing 80 compresses the trigger sleeve spring 53. In figure 5 it can be seen the trigger sleeve connector 51 was moved a distance in proximal direction (see distance between ledges 52 and proximal end of mechanics sleeve 83). The proximal movement of the syringe unit 2 relative to the housing 80 further causes the deflectable cap tongues 11 (figure 2) of the cap 10 to abut against a triangle-shaped protrusion 86 (figure 3) on a distal end of the drive unit housing 80. As a consequence, if the syringe unit 2 is further moved into the drive unit housing 80 the protrusion 86 deflects the cap tongues 11 and thereby releases the snap-fit connection of the cap 10 with the cover sleeve 20. Consequently, the cap 10 is wiped off. This leads to the state shown in figure 5. The controller moves then motor 91 in counter direction to move the plunger rod 40 back the short distance of 1 to 5 mm but without unlocking the syringe unit 2. The syringe unit 2, the trigger sleeve 50 and the gripper sleeve 60 are moved back due to the spring force of the trigger sleeve spring 53.

The autoinjector 1 is now decapped and ready for an injection. The controller displays a corresponding notification on the display.

As a next step the user places the distal end of the cover sleeve 20 onto an injection site and pushes the autoinjector 1 towards the injection site. This causes the cover sleeve 20 to move proximally from the distal covering position into the drive unit housing 80 (push on skin) and compresses the cover sleeve spring 46 which biases the cover sleeve 20 in distal direction via cover sleeve connector 45. Upon push on skin the cover sleeve connector 45 is moved proximally and thus the detection pin is further moved proximally. This further movement is detected by a further TMR position sensor which triggers the controller in the electronic module to start the injection. That means the controller drives the electric motor 91 in dispensing direction and thus rotates the threaded rod 41 and thereby moves the plunger rod 40 in distal direction. As the trigger sleeve spring 53 biases the trigger sleeve connector 45 on the proximal shoulder of the plunger rod 40 (alternatively the trigger sleeve connector 45 is immovably connected to the plunger rod 40) the trigger sleeve 50 is also moved in distal dispensing direction. The distal flange 49 of the plunger rod 40 moves the piston 16 inside the syringe 15 in distal direction and thus dispenses liquid drug through the injection needle out of the syringe 15.

The distal movement of the plunger rod 40 moves the trigger sleeve via trigger sleeve connector 51 in distal direction to its initial position. That means the trigger arms 54 can move radially outwards back into their non-deflected position. However, as the cover sleeve 20 is in a retracted proximal position the locking arms 32 are prevented from being moved back into the locking position.

The controller displays a holding time indicating a time period during that the user has to hold the autoinjector 1 onto the injection side after the injection to ensure that the drug is completely administered and absorbed. The controller may display a down counter informing the user about the remaining holding time. Once the holding time is elapsed a notification is shown to the user that the autoinjector can be removed from the injection site. Alternatively, the end of the holding time may be indicated only by a LED.

When the user removes the autoinjector 1 from the injection site compressed cover sleeve spring 46 can release and thus it can move the cover sleeve 20 back distally into the needle covering position.

During dispensing the trigger sleeve 50 was moved in its initial distal and unlocking position in which the trigger arms 54 are not deflected anymore and thus do not contact the locking arms 32 of the syringe holder 30. That means the locking arms 32 are no longer completely pressed radially inwards and upon movement of the cover sleeve 20 into its covering position the locking arms 32 are able to switch back into their locking position in which the free end of the arms 32 abuts the contact surface inside the cover sleeve 20 thereby locking the cover sleeve 20 in its covering position as in the state shown in figure 4.

Furthermore, as the trigger arms 54 are not deflected anymore and thus do not contact the distal stop surface of the syringe holder 30 the entire syringe unit 2 can be pulled out of the drive unit housing 80 by the user. The user can now discard the syringe unit 2 and the drive unit 3 is ready to be loaded with a new syringe unit.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims.

**LIST OF DESIGNATIONS**

| | | | |
|---|---|---|---|
| 1 | autoinjector | 50 | trigger sleeve |
| 2 | syringe unit | 51 | trigger sleeve connector |
| 3 | drive unit | 52 | ledge |
| 4 | button | 53 | trigger sleeve spring |
| 10 | cap | 54 | trigger arm |
| 11 | deflectable tongues | 55 | arm sloped contact surface |
| 12 | clamps | 56 | free end |
| 15 | prefilled syringe | | |
| 16 | piston | 60 | gripper sleeve |
| 17 | rigid needle shield | 61 | clamping arms |
| 18 | injection needle | 62 | clamp |
| | | 64 | gripper sleeve spring |
| 20 | cover sleeve | 65 | counter release surface |
| 21 | first opening | | |
| 22 | second opening | 80 | housing |
| 23 | indentation | 81 | distal housing insert |
| 25 | label | 82 | contact surface |
| 26 | RFID tag | 83 | mechanics sleeve |
| 27 | release surface | 84 | wall |
| | | 85 | proximal housing insert |
| 30 | syringe holder | 86 | protrusion |
| 31 | longitudinal rail | 91 | electric motor |
| 32 | locking arms | 92 | pinion |
| 33 | ledge | 93 | gear |
| 34 | bearing surface | 94 | battery |
| 40 | plunger rod | | |
| 41 | threaded rod | | |
| 45 | cover sleeve connector | | |
| 46 | cover sleeve spring | | |
| 47 | syringe connector | | |
| 48 | syringe connector spring | | |
| 49 | flange | | |

## Claims

1. A disposable syringe unit (2) for an injection device (1), adapted for releasable attachment to a reusable drive unit (3) of the injection device (1), the syringe unit (2) comprising:
- a prefilled syringe including a reservoir and an injection needle (18) permanently connected to the reservoir and optionally covered by a needle shield in a shipping state;
- a syringe holder (30) for holding the prefilled syringe (15), the syringe holder (30) comprising guiding means (31) and defining a longitudinal axis;
- a cover member (20) guided by the guiding means (31) thereby movable along the longitudinal axis relative to the syringe holder (30) between a covering position in which the injection needle (18) is covered and a retracted position in which the injection needle (18) protrudes from the cover member (20);
- a cap (10) releasably attached to a distal end of the cover member (20) or the syringe holder (30);
**characterized in that**
apart from the prefilled syringe the syringe unit (30) comprises in total not more than three separate parts.

2. The disposable syringe unit (2) according to claim 1, wherein the syringe holder (30), the cover sleeve (20) and the cap (10) are injection-molded thermoplastic plastics parts.

3. The disposable syringe unit (2) according to claim 1 or 2, wherein the syringe is non-movable arranged within the syringe holder (30) after assembly.

4. The disposable syringe unit (2) according to any of claims 1 to 3 , wherein the syringe (15) includes a cylindrical barrel made of glass and an injection needle (18) made of steel inseparably connected to the barrel.

5. The disposable syringe unit (2) according to any of claims 1 to 4, wherein the syringe unit (2) without prefilled syringe is devoid of metallic parts.

6. The disposable syringe unit (2) according to any of claims 1 to 5, wherein the syringe holder (30) includes a radially protruding flexible arm (32) with a free end portion and wherein the cover member (20) includes a stop surface and wherein the free end portion is adapted to engage the stop surface to hold the cover member (20) in the covering position.

7. The disposable syringe unit (2) according to claim 6, wherein the flexible arm (32) is integrally formed in the syringe holder (30).

8. The disposable syringe unit (2) according to claims 6 or 7, wherein the end portion engages the stop surface in a non-deflected position of the arm (32) and the end portion is disengaged from the stop surface in a deflected state of the arm (32) thereby allowing the cover member (20) to be moved out of the covering position.

9. The disposable syringe unit (2) according to any of claims 1 to 8, wherein the cover member (20) includes a machine-readable code (26) attached on a surface of the cover member (20) the code comprising syringe unit specific identification information.

10. The disposable syringe unit (2) according to any of claims 1 to 9, wherein the cover member (20) is a sleeve and wherein the syringe holder (30) is concentrically arranged inside the sleeve.

11. The disposable syringe unit (2) according to claim 10, wherein the sleeve includes a first radial opening (21) wherein the arm (32) of the syringe holder (30) is accessible from the outside through the first opening (21).

12. The disposable syringe unit (2) according to claim 11, wherein the syringe holder (30) includes a radially extending protrusion (33) for holding the syringe unit (2) by the reusable unit (3) and wherein the sleeve includes a second radial opening (22) wherein the protrusion (33) is accessible from the outside through the second opening (22).

13. The disposable syringe unit (2) according to any of claims 10 to 12, wherein the guiding means (31) of the syringe holder (30) includes a longitudinal groove or a longitudinal spline on an outside surface and wherein the sleeve includes on an inside surface the other of the longitudinal groove or the longitudinal spline.

14. Injection device (1) comprising the disposable syringe unit (2) according to any of claims 1 to 13 and the reusable drive unit (3) including a plunger rod (40) and a drive (91) for moving the plunger rod (40) in a dispensing direction to dispense the drug from the syringe (15), wherein the syringe unit (2) is releasably attachable to the drive unit (3).

15. Injection device (1) according to claim 14, wherein the injection device (1) is a semi-reusable autoinjector and wherein the drive comprises an electric motor (91).
